(19) Europäisches Patentamt
European Patent Office
Office européen des brevets

(11) **EP 4 461 224 A1**

(12) **EUROPEAN PATENT APPLICATION**

(43) Date of publication:
**13.11.2024 Bulletin 2024/46**

(21) Application number: **24175147.8**

(22) Date of filing: **10.05.2024**

(51) International Patent Classification (IPC):
**A61B 5/367** (2021.01)    **A61B 5/00** (2006.01)
**A61B 18/14** (2006.01)

(52) Cooperative Patent Classification (CPC):
**A61B 5/6858; A61B 5/367; A61B 5/7267;**
A61B 18/1206; A61B 18/1492; A61B 2018/00267;
A61B 2018/00357; A61B 2018/00577;
A61B 2018/00613; A61B 2018/00839

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: **11.05.2023 US 202318195995**

(71) Applicant: **Biosense Webster (Israel) Ltd.
2066717 Yokneam (IL)**

(72) Inventor: **GOVARI, Assaf
2066717 Yokneam (IL)**

(74) Representative: **Carpmaels & Ransford LLP
One Southampton Row
London WC1B 5HA (GB)**

(54) **SPATIAL CORRELATION TO IDENTIFY VENTRICLE LOCATION OF PATTERN MATCHING**

(57)    A method includes receiving cardiac signals from multiple locations within a ventricle of a heart of a patient. The received signals are compared with reference signals indicative of an arrhythmia. Based on the comparison, a direction is calculated, towards a location that may demonstrate an increased correlation between the received signals and the reference signals. The direction is indicated to a user.

FIG. 4

**Description**

**FIELD OF THE DISCLOSURE**

This disclosure relates generally to electrophysiological (EP) signals, and specifically to evaluation of electrical propagation in the heart.

**BACKGROUND OF THE DISCLOSURE**

Estimation of electrophysiological signals to determine location of a ventricular arrhythmia was previously suggested in the patent literature. For example, U.S. Patent 7,907,994 describes how ventricular tachycardia (VT) signals are induced in a living subject. Pace-mapped signals are then obtained from multiple points within the ventricle, and automatically compared numerically with the induced signals. Recognition of a high degree of cross correlation between the induced signals and one or more of the pace-mapped signals identifies arrhythmogenic foci or pathways, which may then be ablated, so that the arrhythmia becomes non-inducible.

The present disclosure will be more fully understood from the following detailed description of the examples thereof, taken together with the drawings, in which:

**BRIEF DESCRIPTION OF THE DRAWINGS**

Fig. 1 is a schematic, pictorial illustration of a catheter-based electrophysiology (EP) mapping and ablation system, in accordance with an example of the present disclosure;

Fig. 2 illustrates schematically a method to guide a physician to a candidate left ventricle location of arrhythmia, according to an example of the present disclosure;

Fig. 3 illustrates schematically the buildup of a correlation map of a left ventricle while running the guiding method of Fig. 2, according to an example of the present disclosure; and

Fig. 4 is a flow chart that schematically describes the guiding method of Fig. 2, in accordance with an example of the present disclosure.

**DETAILED DESCRIPTION OF EXAMPLES**

OVERVIEW

To characterize an arrhythmia (e.g., a reentry arrhythmia) of a cardiac chamber, such as a left ventricle (LV), a physician may use a multi-electrode mapping catheter to perform electrophysiological (EP) mapping of suspected tissue pathways and circuits within the chamber.

During an EP procedure, if a transient arrhythmogenic event such as an ectopic beat, premature beat, or premature ventricle complex (PVC) occurs, the physician may record the event and then attempt to stimulate repetition of the event with pacing to enable evaluate the origin or cause of the arrhythmogenic event.

For stimulating repetition of the event with pacing, the physician captures pace-mapped signals from multiple points within the ventricle compares the signals that were recorded. Recognition of a high degree of cross correlation between the recorded signal and one or more of the pace-mapped signals indicates successful provoking of the same transient event that is stored. During pacing to identify the mechanism of complex ventricular arrhythmias, the physician samples different areas of the ventricle and performs pattern matching (e.g., electrogram waveform matching between acquired waveforms and a stored pattern of VT-characteristic waveforms) to identify correlations with defined patterns. In one example, pattern matching is performed on an array of electrodes to identify a location of a VT mechanism.

At times, however, the physician has difficulty finding the ventricle area that provides a good correlation between the sampled data and the stored pattern. For example, acquired waveforms from one or more electrodes may not match up well with the pattern. Sometimes the waveforms from most of the channels correlate well with the stored pattern while only one of few channels fail to correlate to the stored pattern. When this happens, it may be particularly difficult for the physician to determine in what direction and/or orientation to move the catheter to improve the correlation for all the channels.

Examples of the present disclosure that are described hereafter provide techniques to guide the physician to the ventricle area that provides a good correlation between the sampled data and the stored pattern. The disclosed technique relies on the benefit of a processor analyzing vast amount of EP data accumulated as the physician moves the catheter inside the cardiac chamber and pace in different places therein.

In one example, the processor applies statistical analysis related to spatial locations of each of the EP cardiac signals captured, to indicate a direction to a ventricle area toward which the physician can move the catheter. Movement in that direction is expected to provide signals with a better correlation with the stored pattern. Examples statistical analysis methods include principal axis analysis, and regression methods. Another statistical analysis used is to check correlation for each channel at each point and then look for maxima.

In another example, the processor applies a machine learning model (ML) includes a loss function that indicates the direction to a ventricle area toward which the physician can move the catheter.

In an example, the loss function comprises a metric of possible directions with relation to (i) LV anatomical targets known to be potentially arrhythmogenic, (ii) analysis zones of ventricle areas having respective average correlations, and (iii) an analysis zone exhibiting a signal with the lowest correlation. At any given location over the ventricle considered by the physician, a processor applying the ML model finds a direction that minimizes the loss function.

An analysis zone may be an area covered by the multi-electrode catheter at any given location during an electrogram acquisition step. The zone may alternatively be defined by an area of a given size and shape suitable for analysis (e.g., a circle of predefined radius that includes a sufficient number of correlation data points).

In embodiments of this disclosure a physician paces cardiac tissue at different locations and for each pacing location, the processor compares the EP signals invoked in response to the pacing with the stored signals. The processor searches for the tissue location with the best (e.g., highest average) correlation. Optionally, correlation of the signal from each channel is required to be above a pre-defined threshold to meet the criteria. Optionally, the best correlation is the location at which the greatest number of channels meet the criteria of having a correlation above the pre-defined threshold with its counterpart stored signal.

The output of the algorithm is an arrow that points the physician in the direction at which the next pacing should be performed. Once the physician follows the directions to reach the area, new EP signals are acquired therein, and the processor calculates the correlation at the new location. The processor may check if the new location is better correlated with the stored pattern by, for example, achieving an average correlation value above a given threshold and/or achieving a minimal correlation value above a given threshold. If the area is deemed arrhythmogenic the physician may ablate tissue therein to eliminate the arrhythmia (e.g., VT).

In some examples, after the ML model guides the physician, the processor constructs a visual aid in the form of a map of the correlation coefficients, so as to visualize the path constructed by the ML model in order to visualize the advancement in accumulation of correlation data. The map acts as a visual assistive tool to guide the physician if and where to ablate.

To construct the map, the processor stores the correlation coefficient at each of a plurality of different locations and for each of the channels and generates a spatial map of the computed correlation coefficients. The correlation coefficient can be an average of all of the correlation coefficients from the different electrodes (weighted or unweighted) inside an analysis zone or may be correlation coefficients from one or more selected electrodes within the analysis zone.

In other examples, instead of an ML model, the processor applies a statistical model (e.g., principal component analysis (PCA)) to find the direction.

SYSTEM DESCRIPTION

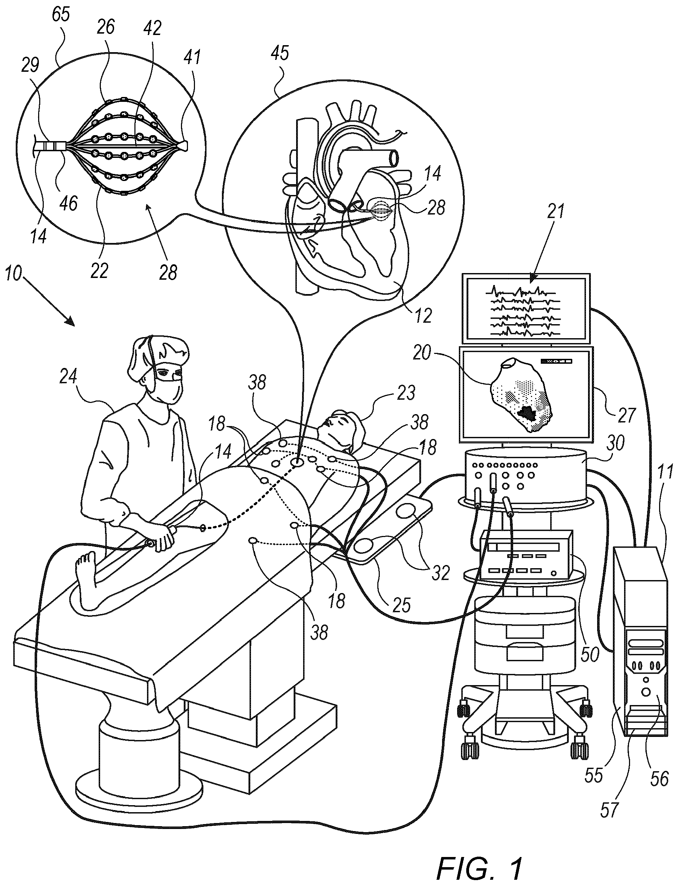

Fig. 1 is a schematic, pictorial illustration of a catheter-based electrophysiology (EP) mapping and ablation system 10, in accordance with an example of the present disclosure.

System 10 includes multiple catheters which are percutaneously inserted by physician 24 through the patient's vascular system into a chamber or vascular structure of a heart 12 (seen in inset 45). Typically, a delivery sheath catheter is inserted into a cardiac chamber, such as the left or right atrium near a desired location in heart 12. Thereafter, a plurality of catheters can be inserted into the delivery sheath catheter so as to arrive at the desired location. The plurality of catheters may include a catheter dedicated for pacing, a catheter for sensing intracardiac electrogram signals, a catheter dedicated for ablating and/or a catheter dedicated for both EP mapping and ablating. An example catheter 14, illustrated herein, is configured for sensing bipolar electrograms. Physician 24 brings a distal tip 28 (also called hereinafter distal end assembly 28) of catheter 14 into contact with the heart wall for sensing a target site in heart 12. For ablation, physician 24 similarly brings a distal end of an ablation catheter to a target site.

As seen in inset 65, catheter 14 is an exemplary catheter that includes a basket distal end 28 one, and preferably multiple, electrodes 26 optionally distributed over a plurality of splines 22 at distal tip 28 and configured to sense IEGM signals. Catheter 14 may additionally include a position sensor 29 embedded in or near distal tip 28 on a shaft 46 of catheter 14, for tracking its position and orientation of distal tip 28. Optionally, and preferably, position sensor 29 is a magnetic-based position sensor including three magnetic coils for sensing three-dimensional (3D) position and orientation. As seen, distal tip 28 further includes an expansion/collapse rod 42 of expandable assembly 28 that is mechanically connected to basket assembly 28 at a distal edge 41 of assembly 28.

Magnetic based position sensor 29 may be operated together with a location pad 25 that includes a plurality of magnetic coils 32 configured to generate magnetic fields in a predefined working volume. Real-time position of distal tip 28 of catheter 14 may be tracked based on magnetic fields generated with location pad 25 and sensed by magnetic based position sensor 29. Details of the magnetic based position sensing technology are described in U.S. Patent Nos. 5,5391,199; 5,443,489; 5,558,091; 6,172,499; 6,239,724; 6,332,089; 6,484,118; 6,618,612; 6,690,963; 6,788,967; 6,892,091.

System 10 includes one or more electrode patches 38 positioned for skin contact on patient 23 to establish a location reference for location pad 25 as well as impedance-based tracking of electrodes 26. For impedance-based tracking, electrical current is directed toward electrodes 26 and sensed at electrode skin patches 38 so that the location of each electrode can be triangulated via electrode patches 38. Details of the impedance-based location tracking technology are described in US Patent Nos. 7,536,218; 7,756,576; 7,848,787; 7,869,865; and 8,456,182.

A recorder 11 displays cardiac signals 21 (e.g., electrograms and respective cardiac tissue positions) acquired with body surface ECG electrodes 18 and intracardiac electrograms acquired with electrodes 26 of catheter 14. Recorder 11 may include pacing capability to pace the heart rhythm and/or may be electrically connected to a standalone pacer.

System 10 may include an ablation energy generator 50 that is adapted to conduct ablative energy to one or more electrodes at a distal tip of a catheter configured for ablation. Energy produced by ablation energy generator 50 may include, but is not limited to, radiofrequency (RF) energy or pulsed-field ablation (PFA) energy, including monopolar or bipolar high-voltage DC pulses, to be used to effect irreversible electroporation (IRE), or combinations thereof.

Patient interface unit (PIU) 30 is an interface configured to establish electrical communication between catheters, electrophysiological equipment, power supply and a workstation 55 to control system 10 operation and receive EP signals from the catheter. Electrophysiological equipment of system 10 may include, for example, multiple catheters, location pad 25, body surface ECG electrodes 18, electrode patches 38, ablation energy generator 50, and recorder 11. Optionally, and preferably, PIU 30 additionally includes processing capability for implementing real-time computations of catheter locations and for performing ECG calculations.

Workstation 55 includes memory 57, processor 56 unit with memory or storage with appropriate operating software loaded therein, and user interface capability. Workstation 55 may provide multiple functions, optionally including (i) modeling endocardial anatomy in three-dimensions (3D) and rendering the model or anatomical map 20 for display on a display device 27, (ii) displaying on display device 27 activation sequences (or other data) compiled from recorded cardiac signals 21 in representative visual indicia or imagery superimposed on the rendered anatomical map 20, (iii) displaying real-time location and orientation of multiple catheters within the heart chamber, and (iv) displaying sites of interest on display device 27 such as places where ablation energy has been applied. One commercial product embodying elements of system 10 is available as the CARTO™ 3 System, available from Biosense Webster, Inc., 31A Technology Drive, Irvine, CA 92618.

In some examples, processor 56 typically comprises a general-purpose computer, which is programmed in software to carry out the functions described herein. The software may be downloaded to the computer in electronic form, over a network, for example, or it may, alternatively or additionally, be provided and/or stored on non-transitory tangible media, such as magnetic, optical, or electronic memory.

This configuration of system 10 is shown by way of example, in order to illustrate certain problems that are addressed by examples of the present disclosure and to demonstrate the application of these examples in enhancing the performance of such a system. Examples of the present disclosure, however, are by no means limited to this specific sort of example system, and the principles described herein may similarly be applied to other sorts of medical systems. For example, other multi-electrode catheter types may be used, such as the OCTARAY™ catheter or a basket catheter.

IDENTIFYING VENTRICLE LOCATION EXHIBITING ARRHYTHMIA

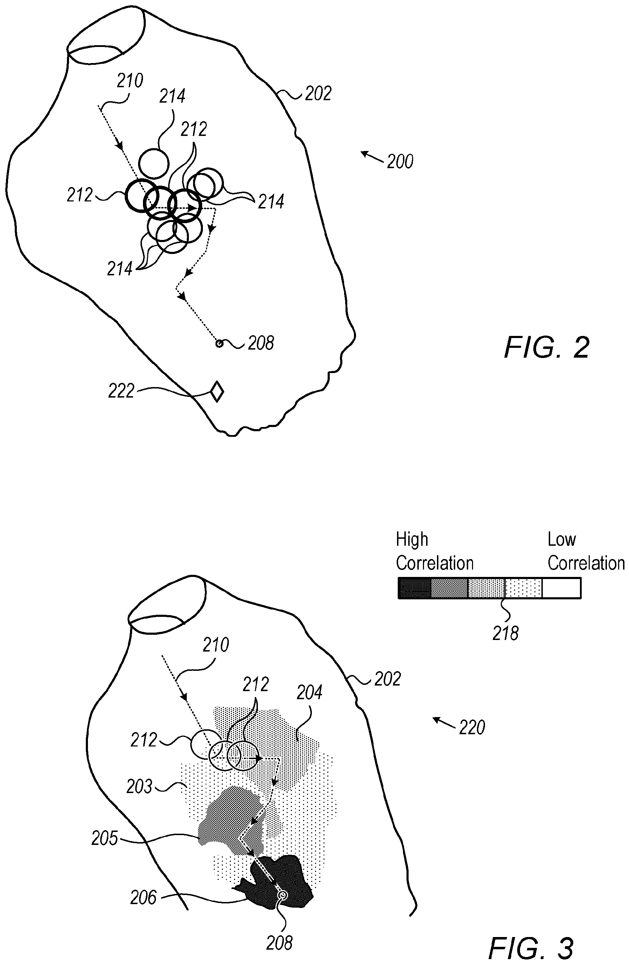

Fig. 2 illustrates schematically a method to guide a physician to a left ventricle location of an arrhythmia, according to an example of the present disclosure. At any given location over the left ventricle that the physician considers, a processor applying a statistical analysis method, or an ML model provides the physician with a direction **d** towards a ventricle area with a better correlation between the sampled data and the stored pattern.

In Fig. 2, given a location on a guiding path 210 over an anatomical surface 202 of an LV (e.g., a location already selected inside an analysis zone 212), a direction **d** toward an area of interest (i.e., exhibiting increased correlation) is considered by deciding between candidate analysis zones 214. In one exmaple, sufficient correlation means that each of the channels of the mapping catheter (e.g., of catheter 14) has a correlation above a defined threshold. In such exmaple, even if one of the channels has a too low correlation the physician needs to move the catheter to improve this. In another exmaple, it is sufficient that the average correlation over channels at a location is above a defined threshold.

As noted above, a machine learning model (ML) can be used to make the decision. The ML model includes a loss function f comprising a metric with relation to (i) directions $v_{ai}$ to anatomical targets (also called known anatomical locations) known to exhibit arrhythmia, (ii) direction $v_{bj}$ to a zone with the highest average correlation among candidate analysis zones 214, and (iii) direction $v_{cj}$ to a location in a analysis zone that demonstrates the lowest correlation within a candidate analysis zone 214, in the case that the correlation at zone 214 is under a given threshold, so as to be considered adversely in the loss function. An example of such a loss function is,

$$f(i; \boldsymbol{d}) = w_{1i}(1 - |\boldsymbol{d} \cdot \boldsymbol{v}_{ai}|) + \sum_j [w_2 |\boldsymbol{d} \cdot \boldsymbol{v}_{bj}| + w_3(1 - |\boldsymbol{d} \cdot \boldsymbol{v}_{cj}|)]$$

where $i$ is an index running on suspected anatomical regions inside the LV (such as the LV apex 222, the LV appendage and others) and $j$ runs over analysis-zones 214. Weights $w_k$ are parameters of the ML model.

At any given current location on path 210, a next direction $\boldsymbol{d}$ toward an area of interest is determined as one that minimizes the non-negative loss function $f(\boldsymbol{d})$. If a location inside an analysis zone 212 is determined as the target of ablation by the physician who was guided to such a zone, the above processor guidance ends. A visual aid can be provided in a form of a correlation map 200, as described below.

GUIDING ASSISTIVE MAP TOOL

Fig. 3 illustrates schematically the buildup of a correlation map 220 of a left ventricle while running the guiding method of Fig. 2, according to an example of the present disclosure. In the shown example, as the ML model guides the physician along path 210 over LV anatomical surface 202, the processor constructs map 200 of the correlation coefficients, so as to visualize path 210 constructed by the ML model, and to visualize the advancement in correlation data in the form of areas 203-206 that are described by a scale 218. The map acts as a visual assistive tool to guide the physician toward region of interest 208 (also called in this disclosure area of interest 208).

METHOD OF GUIDING A PHYSICIAN TO VENTRICLE LOCATION OF ARRHYTHMIA

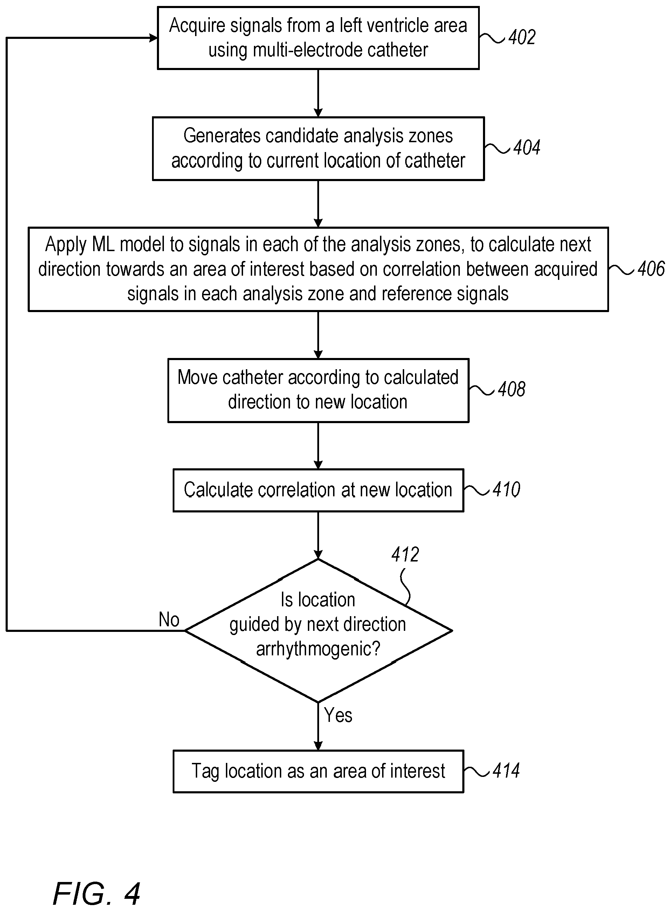

Fig. 4 is a flow chart that schematically describes the guiding method of Fig. 2, in accordance with an example of the present disclosure. The algorithm, according to the presented example, carries out a process that begins with physician 24 acquiring signals from a left ventricle area using multi-electrode catheter 14, at a signal acquisition step 402.

Processor 56 generates candidate analysis-zones 214 according to the current location of the catheter, at analysis zones generation step 404.

In correlation analysis step 406, processor 56 applies the ML model of Fig. 2 on each of analysis zones 214, to calculate next direction $\boldsymbol{d}$ on path 210 toward an area of interest so considered by demonstrating the largest increased correlation.

Next, the physician moves the catheter along the calculated direction to a new location, at a next signal acquisition step 408.

In correlation calculation step 410, processor 56 calculates the correlation at the new location for each of the channels.

In checking the arrival to area of interest step 412, processor 56 checks if the new location guided by the next direction $\boldsymbol{d}$ on path 210 is an area of interest is arrhythmogenic. This can be done by, for example, achieving a value of average correlation above a given threshold and/or achieving a value of minimal correlation above a given threshold.

If the answer is yes, the process ends at a tagging step 414, in which processor 56 tags the current location as an area of interest. If the answer is no, the process returns to step 402.

The example flow chart shown in Fig. 4 is chosen purely for the sake of conceptual clarity. For example, other analysis methods, such as PCA, may be used. Additional steps may be performed, such as receiving indications of the degree of physical contact of the electrodes with diagnosed tissue from a contact force sensor. This and other possible steps are omitted from the disclosure herein purposely in order to provide a more simplified flow chart.

EXAMPLES

Example 1

A method includes receiving cardiac signals (21) from multiple locations within a ventricle of a heart (12) of a patient. The received signals are compared with reference signals indicative of an arrhythmia. Based on the comparison, a direction is calculated, towards a location (208, 222) that may demonstrate an increased correlation between the received signals and the reference signals. The direction is indicated to a user.

Example 2

The method according to example 1, wherein comparing comprises applying a statistical analysis method for estimating the direction.

Example 3

The method according to example 1, wherein comparing comprises applying a machine learning (ML) model comprising a loss function for the direction.

Example 4

The method according to any of examples 1 and 3, and comprising including in the loss function a loss term of direction with regard to direction towards a known anatomical location (222).

Example 5

The method according to any of examples 1 and 3 and 4, wherein calculating the direction comprises finding a direction among directions towards candidate analysis-zones (214), the direction minimizes the loss function.

Example 6

The method according to any of examples 1 through 5, and comprising checking if the location guided to by the direction is an area of interest (208) as arrhythmogenic, and if such tagging the location, and if not receiving cardiac signals from additional multiple locations and, based on analyzing the additional cardiac signals indicating a next direction to a user.

Example 7

The method according to claim 6, wherein checking if the location is an area of interest comprises checking if an average value of correlation over an analysis-zone (214) comprising the location is above a given threshold and/or if a value of minimal correlation in the analysis-zone (214) is above a given threshold.

Example 8

The method according to claim 6, and comprising constructing a map (220) of the correlation, so as to visualize a path (210) that the direction and next directions generate and visualize advancement in correlation data.

Example 9

The method according to claim 1, wherein receiving the cardiac signals comprises receiving one of unipolar and bipolar electrograms acquired using a catheter (14).

Example 10

A system (10) includes an interface (30) and a processor (56). The interface (30) is configured to receive cardiac signals from multiple locations within a ventricle of a heart of a patient. The processor (560 is configured to (i) compare the received signals with reference signals indicative of an arrhythmia, (ii) based on the comparison, calculate a direction towards a location (208, 222) that may demonstrate an increased correlation between the received signals and the reference signals, and (iii) indicate the direction to a user.

It will be appreciated that the examples described above are cited by way of example, and that the present disclosure is not limited to what has been particularly shown and described hereinabove. Rather, the scope of the present disclosure includes both combinations and subcombinations of the various features described hereinabove, as well as variations and modifications thereof which would occur to persons skilled in the art upon reading the foregoing description and which are not disclosed in the prior art.

**Claims**

1. A system (10), comprising:

   An interface (30) configured to receive cardiac signals from multiple locations within a ventricle of a heart of a patient; and
   a processor (560), which is configured to:

      compare the received signals with reference signals indicative of an arrhythmia;
      based on the comparison, calculate a direction towards a location (208,222) that may demonstrate an increased correlation between the received signals and the reference signals; and
      indicate the direction to a user.

2. The system according to claim 1, wherein the processor is configured to compare by applying a statistical analysis method for estimating the direction.

3. The system according to claim 1, wherein the processor is configured to compare by applying a machine learning (ML) model comprising a loss function for the direction.

4. The system according to any of claims 1 and 3, wherein the processor is further configured to include in the loss function a loss term of direction with regard to direction towards a known anatomical location (222).

5. The system according to any of claims 1, 3 and 4, wherein the processor is configured to calculate the direction by finding a direction among directions towards candidate analysis-zones (214), the direction minimizes the loss function.

6. The system according to any preceding claim, wherein the processor is further configured to check if the location guided to by the direction is an area of interest (208) as arrhythmogenic, and if such tag the location, and if not receive cardiac signals from additional multiple locations and, based on analyzing the additional cardiac signals indicating a next direction to a user.

7. The system according to claim 6, wherein the processor is configured to check if the location is an area of interest by checking if an average value of correlation over an analysis-zone comprising the location is above a given threshold and/or if a value of minimal correlation in the analysis-zone is above a given threshold.

8. The system according to claim 6, wherein the processor is further configured to construct a map of the correlation, so as to visualize a path that the direction and next directions generate and visualize advancement in correlation data.

9. The system according to claim 1, wherein the interface is configured to receive the cardiac signals by receiving one of unipolar and bipolar electrograms acquired using a catheter.

FIG. 1

*FIG. 2*

High
Correlation

Low
Correlation

*FIG. 3*

Acquire signals from a left ventricle area
using multi-electrode catheter ~402

Generates candidate analysis zones
according to current location of catheter ~404

Apply ML model to signals in each of the analysis zones, to calculate next
direction towards an area of interest based on correlation between acquired ~406
signals in each analysis zone and reference signals

Move catheter according to calculated
direction to new location ~408

Calculate correlation at new location ~410

412

Is location
No — guided by next direction
arrhythmogenic?

Yes

Tag location as an area of interest ~414

## FIG. 4

Europäisches
Patentamt

European
Patent Office

Office européen
des brevets

# EUROPEAN SEARCH REPORT

**Application Number**

EP 24 17 5147

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 11 564 591 B1 (NARAYAN SANJIV M [US] ET AL) 31 January 2023 (2023-01-31) <br> * Page 1, Lines 16-18; Page 3, Lines 5-11 & Lines 60-65; Page 4, Lines 11-23; Page 7, Lines 22-29; Page 12, Lines 48-51; Page 18, Lines 14-18; Page 23, Lines 21-34; Page 24, Line 41-52; Page 26, Lines 32-36; Page 43, Lines 60-67 and Page 44, Lines 1-4; Page 46, Line 46-51; Page 47, Lines 6-12 & Lines 42-66; Page 53, Lines 14-31; Page 55, Lines 60-67; Page 59, Lines 3-10; claim 1 * | 1-9 | INV.<br>A61B5/367<br>A61B5/00<br>A61B18/14 |
| | ----- | | |
| A | US 2021/259765 A1 (NARAYAN SANJIV M [US]) 26 August 2021 (2021-08-26) <br> * claims 1, 12, 22 * | 1-9 | |
| | ----- | | |

| | |
|---|---|
| | **TECHNICAL FIELDS SEARCHED (IPC)** |
| | A61B |

The present search report has been drawn up for all claims

| Place of search | Date of completion of the search | Examiner |
|---|---|---|
| The Hague | 11 June 2024 | Chacon Caldera, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

.........................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 24 17 5147

This annex lists the patent family members relating to the patent documents cited in the above-mentioned European search report.
The members are as contained in the European Patent Office EDP file on
The European Patent Office is in no way liable for these particulars which are merely given for the purpose of information.

11-06-2024

| Patent document cited in search report | | Publication date | Patent family member(s) | | Publication date |
|---|---|---|---|---|---|
| US 11564591 | B1 | 31-01-2023 | CA | 3237761 A1 | 01-06-2023 |
| | | | US | 11564591 B1 | 31-01-2023 |
| | | | US | 2023200676 A1 | 29-06-2023 |
| | | | WO | 2023096666 A1 | 01-06-2023 |
| US 2021259765 | A1 | 26-08-2021 | AU | 2021224243 A1 | 15-09-2022 |
| | | | CA | 3171471 A1 | 26-08-2021 |
| | | | CN | 115768346 A | 07-03-2023 |
| | | | EP | 4106625 A1 | 28-12-2022 |
| | | | IL | 295739 A | 01-10-2022 |
| | | | JP | 2023517284 A | 25-04-2023 |
| | | | US | 2021259765 A1 | 26-08-2021 |
| | | | WO | 2021168380 A1 | 26-08-2021 |

EPO FORM P0459

For more details about this annex : see Official Journal of the European Patent Office, No. 12/82

**EP 4 461 224 A1**

REFERENCES CITED IN THE DESCRIPTION

**Patent documents cited in the description**

- US 7907994 B
- US 55391199 B
- US 5443489 A
- US 5558091 A
- US 6172499 B
- US 6239724 B
- US 6332089 B
- US 6484118 B
- US 6618612 B
- US 6690963 B
- US 6788967 B
- US 6892091 B
- US 7536218 B
- US 7756576 B
- US 7848787 B
- US 7869865 B
- US 8456182 B